# EUROPEAN PATENT APPLICATION

(11) **EP 4 817 590 A1**
(43) Date of publication of application: **07.10.2026**
(21) Application number: 25168074.0
(22) Date of filing: 02.04.2025
(51) Int. Cl.: A61K 8/06, A61K 8/34, A61K 8/41, A61K 8/42, A61K 8/44, A61Q 19/00

(54) **MOISTURISER COMPOSITIONS**

(71) Applicant: The Boots Company PLC, Nottingham NG2 3AA (GB)
(72) Inventor: JOHNSON, Mark, Nottingham, NG15 8DQ (GB); WILLIAMS, Samantha, Nottingham, NG8 5AY (GB)

(57) **Abstract**

A skincare composition comprising (a) a cationic emulsifier comprising an ester quaternary ammonium cationic emulsifier and/or a non-quaternary ammonium cationic emulsifier, and (b) from 0.01 to 0.6 wt.% of a preservative.

## Description

### Field of the Invention

The invention relates to the field of skin care. More particularly, the invention relates to compositions, methods, and uses thereof for improving skin mildness and reducing skin irritation.

### Background of the Invention

Existing solutions for moisturisation of the skin typically require high levels of ingredients that provide an occlusive film on the surface of the skin to lock in moisture. These products often only provide acute and short-term reduction in the water loss. Typically, such acute remedies are greasy and oily and therefore aesthetically unappealing to consumers. They also require high concentrations of ingredients that are costly to the developer and therefore the cost is often passed onto the customer. Additionally, existing products through their occlusive nature may not be suitable for some skin types, such as for example acne, blemish-prone or sensitive skin types.

Generally, cosmetic emulsion, such as moisturisers, contain a high-water content, and therefore require the addition of preservatives to protect the product from contamination by bacteria, mould and yeast. Preservatives are under increasing scrutiny by consumers as they are perceived as harsh on the skin and potentially harmful. Preservatives are active molecules designed to kill microorganisms, as result they can be harsh on the skin causing skin reactions and irritation, leading to some consumers to become sensitised to specific preservatives. There is therefore a need to remove or minimise preservatives in skincare products to obtain milder formulations that are better tolerated on the skin and suitable to those with the most sensitive of skin.

Skincare compositions are usually based on anionic or non-ionic systems rather than a cationic system and therefore must contain standard levels of preservatives as anionic or non-ionic ingredients do not have inherent antimicrobial activity. Cationic products are common in haircare as conditioners but skincare composition using cationic technology are not common. Some products exist on the market using cationic emulsifiers, but these use older technology based on quaternary ammonium species (quats), which can be slightly irritating to the skin and eyes and persistent in the environment. Cationic ingredients have inherent antimicrobial activity as the positive charge of the cationic binds to the negatively charged bacterial cell membrane, ultimately damaging the membrane and destroying the cell. The inherent antimicrobial activity can therefore help preserve a formulation and bolster protection against other microbes that some preservatives are not effective against.

There is therefore a need for cationic compositions containing reduced amounts of preservatives whilst retaining adequate microbial protection and moisturisation of the skin.

The present invention provides milder skincare composition comprising cationic emulsifier and a reduced amount of preservatives, which are not harsh on the skin, reduce the risk of skin irritation and are suited to those with the most sensitive of skin. The compositions of the present invention also provide effective antimicrobial properties as well as moisturisation of the skin and have improved skin mildness. The compositions of the present invention also use newer cationic technologies (e.g. esterquats and non-quats) that facilitate its biodegradation and have lower toxicity to aquatic life, hence more environmentally friendly compared to older quaternary surfactants.

### Summary of the Invention

The present invention therefore provides in a first aspect a skincare composition comprising (a) a cationic emulsifier comprising an ester quaternary ammonium cationic emulsifier (esterquat cationic emulsifier) and/or a non-quaternary ammonium cationic emulsifier (non-quat cationic emulsifier), and (b) from 0.01 to 0.6 wt.% of preservative, based on the total weight of the composition.

In a second aspect, the present invention provides a use of the skincare composition of the first aspect for a topical cosmetic application to the skin of a subject.

In a third aspect, the present invention provides a use of the skincare composition of the first aspect for moisturising and/or hydrating the skin of a subject.

In a fourth aspect, the present invention provides a use of the skincare composition of the first aspect on skin as a non-therapeutic cosmetic treatment for improving skin mildness and/or preventing skin irritation.

In a fifth aspect, the present invention provides a use of the skincare composition of the first aspect on skin as a non-therapeutic cosmetic treatment for any one or more of the following: improving skin moisture content, hydrating skin, improving the radiance of the skin, improving skin suppleness, reducing skin tightness, improving the healthy visual appearance of the skin, reducing skin dryness, improving the skin barrier function or any combination thereof.

In a sixth aspect, the present invention provides a method comprising applying the skincare composition of the first aspect to the skin of a subject.

In a seventh aspect, the present invention provides a cosmetic method of improving skin mildness and/or reducing skin irritation, the method comprising topically administering the skincare composition of the first aspect to the skin of a subject.

In an eighth aspect, the present invention provides a cosmetic method of reducing skin dryness and/or moisturising and/or hydrating a skin in need thereof, the method comprising topically administering the skincare composition of the first aspect to the skin of a subject.

### Detailed Description of the Invention

The object of the present invention is to improve skin mildness and reduce skin irritation of skincare compositions comprising preservatives.

The present invention relates to a skincare composition comprising (a) a cationic emulsifier comprising an ester quaternary ammonium cationic emulsifier (esterquat cationic emulsifier) and/or a non-quaternary ammonium cationic emulsifier (non-quat cationic emulsifier), and (b) from 0.01 to 0.6 wt.% of preservative, based on the total weight of the composition.

The skincare composition of the present invention is a cosmetic composition. A cosmetic composition is a product designed for use by a consumer and is preferably a skincare cosmetic composition, more preferably a facial skincare cosmetic composition. The skincare composition of the present invention does not include hair conditioning compositions.

Moisturisers are usually based on anionic or non-ionic systems rather than a cationic system so a reduction in preservative levels is not usually possible. The present invention is based on a cationic composition comprising a cationic emulsifier.

The skincare composition of the present invention may be an emulsion. The skincare composition may be in the form of an oil-in-water emulsion. The composition of the present invention may be a cationic emulsion, such as for example a cationic oil-in-water emulsion. The composition of the present invention may be a moisturising cationic moisturiser.

It is typical for preservatives to be incorporated into skincare compositions such as moisturisers at concentrations from 0.7 to 1%. The present inventors have surprisingly found that the antimicrobial activity of the cationic emulsifier can help preserve the formulation and bolster protection against other microbes that preservatives are not effective against and also allows for reduced concentrations of preservatives being incorporated into the skincare composition.

The present invention provides skincare compositions having skin feel and mildness benefits. The present invention also provides skincare compositions that prevent irritation of the skin caused by high levels of preservatives typically used in cosmetic compositions, while still providing a product safe from contamination by bacteria, mould and yeast. The skincare compositions of the present invention are mild to the skin.

The use of cationic emulsifiers can also form cationic compositions (e.g. cationic emulsions) with lamellar structures (lamellar liquid crystals). Said cationic compositions are able to incorporate high levels of humectants and oils without the associated stickiness or tackiness. This is presumably due to these ingredients being incorporated (shielded) into the lamellar structures of the emulsion. The compositions of the present invention comprising a cationic emulsifier are also able to bind to the skin (skin has a negative charge) more efficiently and the lamellar structures can improve the penetration of the actives into the skin. This allows actives of the composition to work more effectively. The lamellar structures can also trap greater oil and water loads in the skin in a long lasting stable format.

The use of cationic emulsifiers also has improved sustainability credentials (natural content & biodegradability) compared to known composition containing quats emulsifiers.

In particular, topical application of the composition of the present invention is useful for improving skin moisture content and/or reducing skin drying and/or reducing transepidermal water loss (TEWL) from the skin's surface and providing short term and long term consistent moisturisation of the skin (including hydrating the skin). Topical application of the composition of the present invention is also useful for improving the healthy visual appearance and/or radiance of the skin.

The skincare composition of the present invention may comprise (a) a cationic emulsifier comprising an ester quaternary ammonium cationic emulsifier (esterquat cationic emulsifier) and/or a non-quaternary ammonium cationic emulsifier (non-quat cationic emulsifier), and (b) from 0.01 to 0.6 wt.% of preservative, based on the total weight of the composition, wherein the a non-quaternary ammonium cationic emulsifier (non-quat) comprises an amino lipid, an amidoamine, or combinations thereof.

The skincare composition of the present invention may comprise (a) a cationic emulsifier comprising an ester quaternary ammonium cationic emulsifier (esterquat cationic emulsifier) and/or a non-quaternary ammonium cationic emulsifier (non-quat cationic emulsifier), and (b) from 0.01 to 0.6 wt.% of preservative, based on the total weight of the composition, wherein the preservative comprises phenoxyethanol.

The skincare composition of the present invention may comprise (a) from 0.05% to 14% of a cationic emulsifier comprising an ester quaternary ammonium cationic emulsifier (esterquat cationic emulsifier) and/or a non-quaternary ammonium cationic emulsifier (non-quat cationic emulsifier), and (b) from 0.01 to 0.6 wt.% of preservative, based on the total weight of the composition, wherein optionally the a non-quaternary ammonium cationic emulsifier (non-quat) comprises an amino lipid, amidoamine, or combinations thereof.

The skincare composition of the present invention may comprise (a) a cationic emulsifier comprising an ester quaternary ammonium cationic emulsifier (esterquat cationic emulsifier) and/or a non-quaternary ammonium cationic emulsifier (non-quat cationic emulsifier), and (b) from 0.01 to 0.6 wt.% of preservative, based on the total weight of the composition, wherein the a non-quaternary ammonium cationic emulsifier (non-quat) comprises amino lipid, amidoamine, or combinations thereof, wherein optionally the preservative comprises phenoxyethanol.

The skincare compositions of the present invention may be employed for purely cosmetic or skincare purposes. So the present invention further relates to a use of the composition of the present invention for the topical application to the skin.

The skincare composition according to the present invention may be used for application to the skin of a subject. The composition of the present invention may be used on any part of the skin, but preferably the composition is used on the face.

The skincare composition of the present invention may be used on skin for the purpose of any one of moisturising skin (including hydrating skin), improving skin moisture content, improving the radiance of the skin, improving skin suppleness, reducing skin tightness, improving the healthy visual appearance of the skin, nourishing skin, improving the skin barrier function, reducing skin dryness, or any combination thereof.

The present invention provides a use of the skincare composition of the present invention for moisturising and/or hydrating the skin of a subject.

The present invention provides a use of the composition of the invention as a non-therapeutic cosmetic treatment to improve the condition of the skin and/or improve skin moisture content and/or reduce skin dryness.

The present invention also provides a use of the skincare composition of the present invention for improving skin mildness or preventing skin irritation in the skin of a subject.

The use of the skincare composition according to the present invention may also improve skin barrier function when applied on a person's skin.

The uses of a composition according to the present invention may reduce transepidermal water loss (TEWL) from the skin's surface by the topical application of the composition of the present invention.

The uses of the present invention comprise the skincare composition described herein, in all aspects and embodiments of the invention.

### Cationic emulsifier

The skincare compositions of the invention comprise a cationic emulsifier.

The cationic emulsifier used may be for use in water-in-oil or oil-in-water emulsions.

In the present invention, the cationic emulsifier does not comprise traditional quaternary ammonium compounds, such as centrimonium chloride (CTAC), behentrimonium chloride (BTAC) or dialkyldimethylammonium salts. The term "traditional quaternary ammonium compounds" does not include, imply or comprise ester-quats or quats with an ester bond in the structure of the cation. CTAC and BTAC are also classed as alkyl-quats, in the present invention, the cationic emulsifier is "alkyl quat-free".

The cationic emulsifier of the skincare compositions of the present invention may be an ester quaternary ammonium cationic emulsifier (esterquat cationic emulsifier) and/or a non-quaternary ammonium cationic emulsifier (non-quat cationic emulsifier). Non-quat or quat-free cationic emulsifiers do not contain a quaternary ammonium compound.

The cationic emulsifier may be an ester quaternary ammonium cationic emulsifier (esterquat), a cationic amino lipid emulsifier, an amidoamine cationic emulsifier or combinations thereof. Esterquats are quaternary ammonium compounds having an ester linkage.

The skincare composition of the present invention comprises a cationic emulsifier, wherein the cationic emulsifier may comprise ester quaternary ammonium compound (esterquat), amino lipid, amidoamine, or combinations thereof.

The cationic emulsifier may comprise beta hydroxy ammonium esters, amino ester lipids (or fatty acid amino esters), fatty acid amido alkyl di alkyl amine, or combinations thereof.

The cationic emulsifier of the skincare compositions of the present invention may comprise distearoylisopropyl dimonium methosulfate, brassicamidopropyl dimethylamine, brassicyl valinate esylate, distearoylethyl dimonium chloride, bis-(isostearoyl/oleoyl isopropyl) dimonium methosulfate, cannabisamidopropyl dimethylamine, stearamidopropyl dimethylamine, brassicyl isoleucinate esylate or combinations thereof. Brassicyl valinate esylate may be used as a mixture with brassica alcohol and brassica glycerides.

Suitable cationic emulsifiers are commercially available under the trademark names TEGO^{®} Care Eco Cat MB from Evonik, AminoSensyl^{™} SC from Inolex, Procondition^{™} 22 from Inolex, Varisoft^{®} EQ 65 MB from Evonik, Varisoft^{®} EQ100 from Evonik, Procondition^{™} Sativa from Inolex, Emulsense^{™} HC from Inolex, Lexamine^{™} S-13 MB from Inolex.

The cationic emulsifier may be present in the skincare compositions of the present invention in an amount by weight of from 0.05% to 14%, such as from 0.1% to 14%, or from 0.15% to 14%, from 0.2% to 14%, from 0.3% to 14%, or from 0.05% to 0.12%, or from 0.1% to 12%, or from 0.15% to 12%, or from 0.2% to 12%, or from 0.3% to 12%, or from 0.05% to 10%, from 0.1% to 10%, or from 0.15% to 10%, or from 0.2% to 10%, or from 0.3% to 10%, or from 0.05% to 8%, or from 0.1% to 8%, or from 0.15% to 8%, or from 0.2% to 8%, or from 0.05% to 6%, or from 0.1% to 6%, or from 0.15% to 6%, or from 0.2% to 6%.

The cationic emulsifier may comprise amino ester lipids or fatty acid amino esters (e.g. brassicyl valinate esylate) which may be present in the composition in an amount by weight of from 1 to 14%, or from 1.5 to 14%, or from 2 to 14%, or from 1 to 12%, or from 1.5 to 12%, or from 2 to 12%, or from 1 to 10%, or from 1.5 to 10%, or from 2 to 10%, or from 1 to 8%, or from 1.5 to 8%, or from 2 to 8%.

The cationic emulsifier may comprise an amidoamine (e.g. brassicamidopropyl dimethylamine) which may be present in the composition in an amount by weight of from 0.05 to 6%, or from 0.05 to 5%, or from 0.0.5 to 4%, or 0.1 to 6%, or from 0.1 to 5.5%, or from 0.1 to 5%, or from 0.1 to 4.5%, or from 0.15 to 6%, or from 0.15 to 5.5%, or from 0.15 to 5%, or from 0.15 to 4.5%, or from 0.2 to 6%, or from 0.2 to 5.5%, or from 0.2 to 5%, or from 0.2 to 4.5%, or from 0.5 to 6%, or from 0.5 to 5.5%, or from 0.5 to 5%, or from 0.5 to 4.5%. or from 0.5 to 3%, or from 0.5 to 2%, or from 1 to 6%, or from 1 to 5.5%, or from 1 to 5%, or from 1 to 4%. or from 1 to 3%, or from 1 to 2%.

The cationic emulsifier may comprise an ester quaternary ammonium compound (esterquat) (e.g. distearoylisopropyl dimonium methosulfate) which may be present in the composition in an amount by weight of from 1 to 6%, or from 1 to 5.5%, or from 1 to 5%, or from 1 to 4.5%, or from 1.5 to 6%, or from 1.5 to 5.5%, or from 1.5 to 5%, or form 1.5 to 4.5%, or from 2 to 6%, or from 2 to 5.5%, or from 2 to 5%, or from 2 to 4.5%, or from 3 to 6%, or from 3 to 5.5%, or from 3 to 5%, or from 3 to 4.5%.

### Preservatives

Preservatives are commonly found in cosmetic emulsions, as they contain significant amounts of water. Preservatives are active molecules that prevent microbial grown and may cause skin irritation and sensitivity.

The skincare compositions of the invention may comprise preservatives such as phenoxyethanol, 2-bromo2-nitropropane-1,3-diol (bronopol, which is available commercially under the trade name Myacide RTM), benzyl alcohol, diazolidinyl urea, imidazolidinyl urea, methyl paraben, phenoxy ethanol, ethyl paraben, propyl paraben, sodium methyl paraben, sodium dehydroacetate, polyhexamethylenebiguanide hydrochloride, sodium benzoate, chlorhexidine digluconate, isothiazolone, sodium propyl paraben or combinations thereof.

The skincare compositions of the invention may comprise phenoxyethanol.

The preservative may be present in the skincare composition of the present invention in an amount by weight of from 0.01% to 0.6%, such as from 0.05% to 0.6%, or from 0.1% to 0.6%, or from 0.2% to 0.6%, 0.01% to 0.5%, or from 0.05% to 0.5%, or from 0.1% to 0.5%, or from 0.2% to 0.5%, or from 0.01% to 0.4%, or from 0.05% to 0.4%, or from 0.1% to 0.4%, or from 0.2% to 0.4%, or from 0.01% to 0.3%, or from 0.05% to 0.3%, or from 0.1% to 0.3%, or from 0.2% to 0.3%, or from 0.01% to 0.2%, or from 0.05% to 0.2%, or from 0.1% to 0.2%.

The skincare compositions of the invention may further comprise one or more preservative boosters. Said preservative boosters may comprise caprylyl glycol and/or ethylhexylglycerin. Caprylyl glycol and ethylhexylglycerin are also known as humectants, but in the present invention these compounds are used as preservative boosters.

The skincare composition may comprise caprylyl glycol and ethylhexylglycerin which may be present in the composition in an amount by weight of from 0.01 to 0.3%, such as from 0.05% to 0.3%, or from 0.1% to 0.3%, or from 0.01% to 0.2%, or from 0.05% to 0.2%, or from 0.1% to 0.2%, or from 0.01% to 0.1%, or from 0.05% to 0.1%.

The skincare compositions of the invention may comprise phenoxyethanol, caprylyl glycol and ethylhexylglycerin.

### Emulsions

The composition of the present invention may be aqueous or non-aqueous and comprise of a single-phase system or multiple phase system. Theskincare composition may include but is not limited to liquids, gels, balms, oils or solids. Single or multiple phase compositions are envisaged. Multiple phase systems include but are not limited to microemulsions, emulsions, and products with discrete separate phases. The skincare composition of the present invention may be an emulsion. Emulsions include water-in-oil, oil-in-water emulsions and multiple emulsions (water in oil in water or oil in water in oil for example). Products with discrete separate phases include bi or triphasic systems where the individual water or oil phases can be visibly seen.

Where the skincare composition of the invention is an emulsion, it comprises an oil and a water phase.

The composition of the present invention may be an oil-in-water emulsion, for example a cationic oil-in-water emulsion. Oil in water emulsions are preferred by consumers as they have a lighter feel on the skin due to them being water based.

The oil in water emulsion may therefore comprise at least 40% by weight water, such as at least 45%, at least 50% or at least 90% by weight water. The oil in water emulsion of the present invention may comprise 90% by weight water or less, such as 85% or less, 80 % or less, or 75% by weight or less. The emulsion may comprise 50% to 95% by weight of the emulsion of water, such as from 50% to 90 % by weight, or from 50% to 85 % by weight, or from 50% to 80 % by weight, from 55% to 95 % by weight, or from 55% to 90 % by weight, or from 55% to 85 %, from 55% to 80 % by weight of water.

The water may comprise de-ionized water, purified water, natural spring water, rose water or the like. Mixtures of more than one of these may also be used. In one embodiment de-ionized or purified water may be used.

It will be appreciated that the water phase may comprise further components as part of a cosmetic emulsion. The water phase may comprise additional components such as humectants, pH adjusters, thickeners, active ingredients such as peptides or other anti-ageing ingredients, and combinations thereof.

The oil phase of an emulsion can be provided by any suitable oily component. Suitable oils for the oil phase may comprise for example: a) hydrocarbon oils, such as paraffin or mineral oils; b) waxes, such as beeswax or paraffin wax; c) natural oils, such as sunflower oil, apricot kernel oil, shea butter or jojoba oil; d) silicone oils, such as dimethicone, silicone elastomer, cyclomethicone or cetylidimethicone; e) fatty acid esters and ethers, such as isopropyl palmitate or isopropyl myristate and polypropylene glycol-15 stearyl ether; f) fatty alcohols, such as brassica alcohol, behenyl alcohol, cetyl alcohol, cetearyl alcohol or stearyl alcohol; or g) mixtures thereof, for example, the blend of waxes available commercially under the trade name Cutina (BASF).

The emulsion may comprise at least 0.1 wt.% of an oil phase. For example, the emulsion may comprise at least 3 wt.% of oil phase, such as at least 5 wt.%, or at least 10 wt.%. The emulsion may comprise 0.1 wt.% to 55% wt.% of oil phase. In one embodiment, emulsion may comprise 0.1 wt.% to 50% wt.% of oil phase, more preferably from 0.1 wt.% to 40 wt.%, or from 0.1 wt.% to 30 wt.%, or from 1 wt.% to 55 wt.%, or from 1 wt.% to 50 wt.%, or from 1 wt.% to 40 wt.%, or from 1 wt.% to 30 wt.%, or from 3 wt.% to 55 wt.%, or from 3 wt.% to 50 wt.%, or from 3 wt.% to 40 wt.%, or from 3 wt.% to 30 wt.%, or from 5 wt.% to 55 wt.%, or from 5 wt.% to 50 wt.%, or from 5 wt.% to 40 wt.%, or from 5 wt.% to 30 wt.%, or from 5 wt.% to 20 wt.%, or from 5 wt.% to 10 wt.% of oil phase.

The oil phase of the emulsion may comprise a combination of oil, wax or butter. Waxes and butters are hydrocarbons that consist of long aliphatic alkyl chains and may include aromatic groups. They are generally lipophilic and typically solid or malleable at room temperature. Melting points vary depending on the alkyl chain, chain length and associations. Silicone waxes are a type of suitable wax based on alkylmethylsiloxane. The wax may be a fatty alcohol wax (e.g. Cetearyl alcohol). Oils are typically lipophilic and liquid at room temperature with lower molecular weights than waxes. Where present, wax or butter may be present at up to 30% by weight of the oil phase of the emulsion. The oil phase may contain wax or butter at levels of up to 20% by weight of the oil phase of the emulsion. The oil phase may contain wax or butter at levels of up to 10% by weight of the oil phase of the emulsion, such as from 1 to 10%, or from 1 to 8% by weight of the oil phase of the emulsion.

The composition of the present invention may be in the form of an oil-in-water emulsion wherein the oil phase of the emulsion may comprise a fatty alcohol wax (e.g. Cetearyl alcohol).

The oil phase may comprise silicone oils which are silicone elastomers. Suitable for use herein are silicone elastomers which can be emulsifying or non-emulsifying crosslinked siloxane elastomers or combinations thereof. No specific restriction exists as to the type of curable organopolysiloxane composition that can serve as starting material for the crosslinked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon- bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane and condensation-curing organopolysiloxane compositions which cure in the presence of an organotin compound or a titanate ester. Preferred silicone elastomers include dimethicone crosspolymer and Polysilicone-11.

The oil phase of the emulsion may comprise a triglyceride. The triglyceride may be a C3 to C16 triglyceride for example a C6 to C12 triglyceride. Examples include tripalmitin and caprylic/capric triglyceride. In one example the triglyceride may be caprylic/capric triglyceride.

### Other components

Th skincare composition may comprise cosmetically acceptable components. The skilled person is able to identify cosmetically acceptable components, and these are well known in the field of the invention.

In one embodiments, the skincare composition of the present invention may further comprise one or more antioxidant agents selected from a) ascorbic acid its salts, esters, glucosides and glucosamines, particularly sodium ascorbyl phosphate, magnesium ascorbyl phosphate, ascorbyl palmitate and ethyl ascorbic acid b) vitamin E (tocopherol) and its esters, particularly tocopheryl acetate, as well as Dimethyl methoxy chromanol which is a synthetic analogue of gamma tocopherol, available from Lipotec S.A. polygon Industrial Camri Ral, under the tradename Lipochroman-6 c) herbal extracts (not including Annona, ginseng and Trifolium extracts), particularly gingko biloba, such as that available under the trade name "Gingko Biloba Leaf Powder" from Univar PLC, morus alba, such as that available under the trade name "Mulberry Concentrate" from Solabia, origanum vulgare, such as that available under the trade name "Pronalen Origanum HSC" from S Black Ltd, birch extract such as those available from Cosmetochem (U. K.) Ltd under the trade names "Super Herbasol Extract Birch" and "HP Herbasol Betula" and those available from Blagden Chemicals under the tradenames "Phytelene of Birch" and "Aqueous Spray Dried Birch", camellia sinensis, such as that available under the trade name "Herbal Extract Green Tea 75% Solids" from Nichimen Europe, rosmarinus officinalis, such as that available under the trade name "Pronalen Rosemary" from S. Black, Acerola cherry powder, such as that available as Acerola PE from Gee Lawson, Emblica extract sold under the tradename Emblica^{™} by Merck Speciality chemicals, and Grape Seed oil, such as that available from Chesham Chemicals Limited.

The skincare composition of the present invention may comprise one or more antioxidant agents selected from green tea extract (e.g. Camellia sinensis leaf extract), Quercus petraea fruit extract (such as that sold under the trade name of Phytessence French Oak^{™} from Croda) and ferulic acid (also known as hydroxy cinnamic acid).

The skincare composition of the present invention may further comprise one or more antioxidant agents selected from flavonoid species, including flavones, flavonols, flavanones, flavanols, anthrocyanidins and isoflavonoids; phenoic acid species; stilbenes; lignans or combinations thereof, which provides an antioxidant benefit. The antioxidant agent may also be a plant extract or derivative thereof. Plants provide a rich and cheap source of antioxidant agents and are therefore an efficient source of said agents. Similar actives can be also prepared synthetically and as such are analogues. The term "antioxidant agent" is also intended to cover said analogues. The plant extract may be selected from the group consisting of essential oils, extracts from leaves, extracts from stems, extracts from petals, extracts from seeds, extracts from roots, extracts from pollen, and combinations thereof.

The antioxidant agent(s) may be selected from the group consisting of dimethylmethoxy chromanol (such as that sold under the trade name of LIPOCHROM AN^{®} Molecule from Lipotec), Myrtus communis leaf extract (such as that sold under the trade name of DEXOSINE BIO^{®} from Silab), Camellia japonica extract (e.g., a Camellia japonica flower extract such as that sold under the trade name of RedSnow^{®} from Clariant. The extract may be obtained from the leaves of Camellia japonica. The extract may be obtained from the roots of Camellia japonica. Camellia japonica flowers may be collected on Jeju island and may contain Protocatechuic acid (200ppm), emblica extract (e.g. Phyllanthus emblica fruit extract (also known as Emblica officinalis (Gooseberry) fruit extract)), ginkgo extract (e.g. Ginkgo biloba leaf extract), Argania spinosa leaf extract (such as that sold under the trade name of Arganyl^{™} from BASF), Pinus pinaster bark extract (such as that sold under the trade name of Pycnogenol), Resveratrol (such as that sold under the trade name of REGU^{®}-FADE), and combinations thereof.

The amounts of the antioxidant agents used in the skincare compositions are expressed as dry weights, as understood by a man skilled in the art. The total amount of antioxidant agents optionally present in the skincare compositions may range from 0.005% to 10% by weight, preferably 0.01% to 5%, most preferably 0.05% to 1.0% by weight of the total weight of the skincare composition.

The skincare compositions of the invention may further comprise one or more ceramides. Ceramides are a family of waxy lipid molecules. A ceramide is composed of sphingosine and a fatty acid.

The skincare composition of the invention may comprise at least one ceramide. The at least one ceramide may comprise ceramide 1 (ceramide EOP), ceramide 2 (ceramide NG), ceramide 3 (ceramide NP) and/or ceramide 6 (ceramide AP). The composition preferably comprises ceramide 3 (ceramide NP), also known as N-oloeoyl-4-hydroxysphinganine. The composition preferably comprises ceramide 6 (ceramide AP).

The skincare composition may comprise at least two ceramides, such as ceramide 3 and ceramide 6. The composition may comprise at least 3 ceramides, such as ceramide 1, ceramide 3, and ceramide 6.

The skincare compositions of the invention may optionally comprise a skin conditioning agent. Said skin conditioning agents may preferably be selected from the group consisting of humectants, emollients, moisturisers, or mixtures thereof. Where present, they are preferably present at a level of from 0.01% to 20% by weight, more preferably from 0.1% to 10% by weight, most preferably from 0.5% to 7% by weight of the skincare composition.

Preferred skin conditioning agents are selected from the group consisting of guanidine, urea, glycolic acid and glycolate salts, salicylic acid, lactic acid and lactate salts, aloe vera, shea butter, polyhydroxy alcohols, such as sorbitol, mannitol, xylitol, erythritol, glycerol, hexanetriol, butanitriol, (di) propylene glycol, butylene glycol, hexylene glycol, polyethylene glycol, sugars (e.g. fructose, glucose, xylose, honey, mannose, xylose), gluconodeltalactone, and starches and their derivatives, pyrrolidone, carboxylic acid, lactamide monoethanolamine, acetamide monoethanolamine, panthenol, allantoin and mixtures thereof.

The skincare composition of the present invention comprises humectant comprising one or more polyols. The polyol may be a diol, a triol or a combination thereof. The skincare composition of the present invention may comprise a humectant that is a diol and/or a triol. The composition may comprise a diol selected from the group consisting of pentylene glycol, butylene glycol, di-propylene glycol, propanediol, hexenediol, butylene glycol, propylene glycol, isoprene glycol, dipropylene glycol, pentylene glycol, hexylene glycol, polypropylene glycol, butylene glycol, polyethylene glycol, sorbitol, glucitol, mannitol, hydroxypropyl sorbitol, erythritol, threitol, pentaerythritol, xylitol. The composition may comprise a triol selected from the group consisting of hexanetriol, glycerol (glycerin), ethoxylated glycerin, propoxylated glycerin and mixtures thereof.

The humectant may comprise propylene glycol, also known as propanediol, butylene glycol, glycerin and/or panthenol.

The humectant in the composition of the present invention may comprise glycerin (e.g. from 3 to 22 wt.%, or from 5 to 22 wt.%, from 10 to 22 wt.%, from 3 to 20 wt.%, or from 5 to 20 wt.%, from 10 to 20 wt.%, from 3 to 18 wt.%, from 5 to 18 wt.%). The skincare composition may comprise propylene glycol, also known as propanediol (e.g., 0.3 to 2 wt.% propylene glycol); butylene glycol (e.g., 0.1 to 0.5 wt.% butylene glycol); glycerin (e.g. 3 to 20 wt.% glycerin); and/or panthenol (e.g., 0.1 to 0.9 wt.% panthenol). The composition may comprise glycerin (e.g. 3 to 20 wt.% glycerin).

The skincare compositions of the invention may comprise one or more vitamins. The composition of the present invention may comprise vitamin B (e.g., vitamin B1 to vitamin B12) or a vitamin B derivative (e.g., a derivative of vitamin B1 to B12, such as a vitamin B3). The composition of the present invention may comprise a vitamin B3 compound, such as niacinamide. Vitamin B3 and niacin, are the common names for nicotinic acid. The physiologically active form of niacin is niacinamide. Niacin and niacinamide (nicotinamide or nicotinic acid amide) function in the body as a component of two coenzymes: nicotinamide adenine dinucleotide (NAD/NAD+) and nicotinamide adenine dinucleotide phosphate (NADP).

The compositions of the present invention preferably may comprise one or more vitamin B3 compounds. Preferred vitamin B3 compounds are niacinamide and tocopherol nicotinate. Niacinamide is more preferred. Salts of the vitamin B3 compound are also useful herein. Non-limiting examples of salts of the vitamin B3 compound useful herein include organic or inorganic salts, such as inorganic salts with anionic inorganic species (e.g., chloride, bromide, iodide, carbonate, preferably chloride), and organic carboxylic acid salts (including mono-, di- and tri- C1-C18 carboxylic acid salts, e.g., acetate, salicylate, glycolate, lactate, malate, citrate, preferably monocarboxylic acid salts such as acetate). The vitamin B3 compound may be included as the substantially pure material, or as an extract obtained by suitable physical and/or chemical isolation from natural sources. The vitamin B3 compound is preferably substantially pure, more preferably essentially pure.

The Vitamin B or vitamin B derivative (e.g. vitamin B3 compound, such as niacinamide) may be present in the composition in an amount by weight of at least 0.01%, at least 0.05%, at least 0.1%, at least 0.5%, at least 1%, at least 2%, at least 3%, at least 4%; and/or the vitamin B or vitamin B derivative (e.g., vitamin B3 compound such as niacinamide) may be present in the composition in an amount by weight of 5% or less, 4% or less, 3% or less, 2% or less, 1% or less, 0.5% or less, 0.1% or less, or 0.05% or less. Where more than one vitamin B/vitamin B derivative is present, the values apply to the total amount of vitamin B or vitamin B derivative.

In particular, the vitamin B or vitamin B derivative (e.g., vitamin B3 compound such as niacinamide) may be present in the composition in an amount by weight of from 0.1 to 5%, such as from 0.1 to 4%, or from 0.1 to 3.5%, or from 0.1 to 3%, or from 0.1 to 2.5%, or from 0.1 to 2%, or form 0.5 to 4%, or from 0.5 to 3.5%, or from 0.5 to 3%, or from 0.5 to 2.5%, or form 1 to 4%, or from 1 to 3.5%, or from 1 to 3%, or from 1 to 2.5%, or form 1.5 to 4%, or from 1.5 to 3.5%, or from 1.5 to 3%, or from 1.5 to 2.5%, or from 1.5 to 2%.

The compositions may comprise ascorbates, for example vitamin C, vitamin C derivatives, ascorbic acid, ascorbyl glucoside, ascorbyl palmitate, magnesium ascorbyl phosphate, sodium ascorbyl phosphate and ethyl ascorbic acid.

In some embodiments the skincare compositions may comprise vitamin K, vitamin K derivatives, vitamin H, vitamin D, vitamin D derivatives and mixtures thereof. In an alternative embodiment of the present the skincare compositions comprise vitamin E, vitamin E derivatives such as tocopherol and tocopheryl acetate, and provitamins thereof, such as panthenol and mixtures thereof.

In a further embodiment, the skincare compositions of the invention comprise retinoid compounds, including retinoic acid, retinaldehyde, retinol and derivatives thereof. In one embodiment the skincare compositions comprise retinyl palmitate, retinyl acetate, retinyl retinoate, retinyl proprionate, retinyl ascorbate, retinyl linoleate, retinyl retinoate, retinyl sunflowerseedate and mixtures thereof.

The vitamin compounds may be included as the substantially pure material, or as an extract obtained by suitable physical and/or chemical isolation from natural (e.g. plant) sources. In one embodiment, when vitamin compounds are present in the skincare compositions of the instant invention, the skincare compositions comprise from about 0.0001% to 50%, more preferably from 0.001% to 10%, still more preferably from 0.01% to 8%, and still more preferably from 0.1% to 5%, by weight of the skincare composition, of the vitamin compound.

The skincare compositions of the invention may optionally comprise a sunscreen component. The sunscreen may comprise organic or inorganic sun filters or a combination of the two. Suitable inorganic sun filters include those selected from the group consisting of microfine titanium dioxide, microfine zinc oxide, boron nitride and mixtures thereof. Suitable organic sunscreens include those selected from the group consisting of: a) p-aminobenzoic acids, their esters and derivatives (for example, 2ethylhexyl p-dimethylaminobenzoate), b) methoxycinnamate esters (for example, 2-ethylhexyl p-methoxycinnamate, 2-ethoxyethyl p-methoxycinnamate or a, p-di- (p-methoxycinnamoyl)-a'- (2ethylhexanoyl)-glycerin, c) benzophenones (for example oxybenzone), d) dibenzoylmethanes such as 4-(tert-butyl)-4'-methoxydibenzoylmethane, e) 2-phenylbenzimidazole-5 sulfonic acid and its salts, f) alkyl-ss, ss-diphenylacrylates for example alkyl a-cyano-ss, ss-diphenylacrylates such as octocrylene, g) triazines such as 2,4,6-trianilino-(p-carbo-2-ethyl-hexyl-1-oxi)-1, 3,5-triazine, h) camphor derivatives such as methylbenzylidene camphor and i) mixtures thereof. Other preferred sunscreen ingredients include those selected from the group consisting of homosalate, Ethylhexyl salicylate, Diethylhexylbutamido triazone, Bis-ethylhexyloxyphenol methoxyphenyl triazine, Diethylamino hydroxybenzoyl hexyl benzoate, Butyl methoxydibenzoylmethane, Methylene bis-benzotriazoyl tetramethylbutylphenol, Polysilicone-15 and mixtures thereof. A sunscreen agent is optionally present in an amount from 0.1 to 10% by weight of the skincare composition.

The composition of the present invention may comprise saccharide isomerate. Saccharide isomerate consists mainly of glucose, fructose mannose and galactose.

Saccharide isomerate mimics NMF (Natural Moisturising Factor) found in human skin and is derived from plant based glucose. Saccharide isomerate is commercially available under the trademark name PENTAVITIN^{®} from DSM Nutritional Products Ltd.

The saccharide isomerate may be present in the composition in an amount by weight of from 0.05% to 1%, such as from 0.05% to 0.95%, or from 0.05% to 0.85%, or from 0.05% to 0.80%, or from 0.05% to 0.75%, from 0.05% to 0.7%, or from 0.05% to 0.65%, or from 0.05% to 0.6%, or from 0.05% to 0.55%, or from 0.05% to 0.5%, or from 0.1% to 0.85%, from 0.1% to 0.80%, or from 0.1% to 0.75%, or from 0.1% to 0.7%, or from 0.1% to 0.65%, or from 0.1% to 0.6%, or from 0.1% to 0.55%, or from 0.1% to 0.5%, or from 0.2% to 0.85%, from 0.2% to 0.80%, or from 0.2% to 0.75%, or from 0.2% to 0.7%, or from 0.2% to 0.65%, or from 0.2% to 0.6%, or from 0.2% to 0.55%, or from 0.2% to 0.5%, or from 0.4% to 0.85%, from 0.4% to 0.80%, or from 0.4% to 0.75%, or from 0.4% to 0.7%, or from 0.4% to 0.65%, or from 0.4% to 0.6%, or from 0.4% to 0.55%, or from 0.4% to 0.5%.

The composition of the present invention may comprise a peptide. Peptides are defined as compounds comprising an uninterrupted sequence of amino acids. Preferred peptides are selected from the group consisting of dipeptides, tripeptides, tetrapeptides, pentapeptides and mixtures thereof.

The peptide may comprise synthetic peptide that inhibits the enzymes plasmin and urokinase.

The peptide may comprise an amidino benzylamine derivative. The peptide may be selected from amidinobenzyl benzylsulfonyl D-seryl homophenylalaninamide acetate (ABSHA), benzylsulfonyl-D-Ser-lys-(4-amidino-benzylamide), benzylsulfonyl-D-Ser-Gly-4-amidino-benzylamide and benzylsulfonyl-D-Ser-Ala-4- amidino-benzylamide.

The peptide may comprise or consist of amidinobenzyl benzylsulfonyl D-seryl homophenylalaninamide acetate (ABSHA). Amidinobenzyl benzylsulfonyl D-seryl homophenylalaninamide acetate (ABSHA) is commercially available under trade name SYN-UP^{®} from DSM Nutritional Products Ltd.

The peptide (e.g., ABSHA) may be present in the composition in an amount by weight of from 0.0001 to 0.0004% (e.g., from 1ppm to 4ppm), such as from 0.00015 to 0.0004%, or from 0.0002 to 0.0004%, or from 0.00025 to 0.0004%, or from 0.0001 to 0.00035%, or from 0.00015 to 0.00035%, or from 0.0002 to 0.00035%, or from 0.00025 to 0.00035%, or from 0.0001 to 0.0003%, or from 0.00015 to 0.0003%, or from 0.0002 to 0.0003%, or from 0.00025 to 0.0003%, or from 0.0001 to 0.00025%, or from 0.00015 to 0.00025%, or from 0.0002 to 0.00025%.

The peptides may be selected from the group consisting of dipeptides, tripeptides, tetrapeptides, pentapeptides, hexapeptides and mixtures thereof.

The skincare compositions of the invention may comprise thickeners, viscosity modifying agents and/or gelling agents, such as guar gum, glucomannan, sclerotium gum, hydroxyethyl cellulose, caesalpinia spinosa gum, guar hydroxypropyltrimonium chloride, polyquaternium-37, hydroxypropyl guar, hydroxyalkyl guar, acrylic acid polymers e.g., available commercially under the trade name Carbopol, Ultrez or Novethix (Lubrizol) or Sepigel, Sepiplus and Simulgel (Seppic). Modified celluloses may also be added, for example hydroxyethylcellulose available commercially under the trade name Natrosol (Hercules) or hydroxypropylmethyl cellulose, amine oxides, block polymers of ethylene oxide and propylene oxide (for example, those available from BASF Wyandotte under the trade name "Pluronic"RTM), PVM, MA, or a decadiene crosspolymer (available under the trade name Stabilez 60), ethoxylated fatty alcohols, salt (magnesium chloride, sodium chloride), Aristoflex AVC (Clariant), phthalic acid amide, xanthan gum, sodium polyacrylate, polyvinyl alcohols, fatty alcohols and alkyl galactomannans available under the trade name N-Hance from Hercules, suitably in an amount of from 0.1% to 10% by weight of the skincare composition.

Sequestering agents may be added to the skincare compositions, such as ethylenediamine tetraacetic acid and salts thereof, suitably in an amount of from 0.005% to 0.5% by weight of the skincare composition.

The skincare compositions may also comprise suitable, cosmetically acceptable diluents, carriers and/or propellants such as dimethyl ether.

The skincare compositions of the invention may also include pearlising agents such as stearic monoethanolamide and/or mica, suitably in an amount of from 0.01% to 10% by weight of the skincare composition.

The skincare compositions of the invention may include perfumes. **In** some embodiments the perfumes are present in an amount of from 0.01% to 2% by weight of the skincare compositions, as may water soluble dyes such as tartrazine, suitably in an amount of from a trace amount (such as 1 x 10⁻⁵ %) to 0.1 % by weight of the skincare composition.

The compositions of the invention may also include pH adjusting agents such as sodium hydroxide, aminomethyl propanol, calcium gluconate, aspartic acid, triethanolamine, suitably in an amount of from 0.01 % to 10% by weight of the skincare composition. The skincare compositions may be buffered by means well known in the art, for example by use of buffer systems comprising succinic acid, citric acid, lactic acid, and acceptable salts thereof, phosphoric acid, mono-or disodium phosphate and sodium carbonate. Suitably, the skincare composition may have a pH between 3 and 10, preferably between 4 and 8, more preferably from 4.5 to 6.

### Methods and processes

The methods of the present invention may comprise applying on a person's skin the skincare compositions of the present invention.

The present invention relates to a method comprising applying the skincare compositions according to the present invention to the skin of a subject.

The present invention also relates to a method of improving skin mildness and/or reducing skin irritation, the method comprising topically administering the skincare composition of the present invention to the skin of a subject.

The invention also relates to a cosmetic method of moisturising and/or hydrating a skin in need thereof, the method comprising topically administering the skincare composition according to the present invention to the skin of a subject.

The present invention also provides a cosmetic method of reducing skin dryness, the method comprising topically administering the skincare composition according to the present invention to the skin of a subject.

The invention further concerns a cosmetic method for improving the skin barrier function, the method comprising topically administering the skincare compositions according to the present invention to the skin of a subject.

The invention further relates to a method of reducing transepidermal water loss (TEWL) from the skin's surface, the method comprising the topical application of the skincare composition of the present invention to the skin of a subject.

The invention further relates to a method of improving the radiance of the skin and/or improving the healthy visual appearance of the skin, the method comprising the topical application of the skincare composition according to the present invention to the skin of a subject.

### Definitions

Unless otherwise indicated, a reference to the composition of the invention is a reference to the compositions of all aspects and embodiments of the invention.

All percentages provided herein are percentages by weight of the composition unless otherwise specified.

### Examples

The present invention is further described by the following examples.

### Exemplary Formulations:

In the following exemplary formulations (examples 1-3), the active levels of components are shown. Other components as discussed in the description may be selected by the skilled person such that the formulation totals 100wt.%

### Example 1 - Cationic moisturiser (distearoylisopropyl dimonium methosulfate)

| Material | wt.% |
|---|---|
| Glycerin | 10.00 |
| Water | 61.10 |
| Phenoxyethanol | 0.40 |
| Caprylyl glycol & ethylhexylglycerin | 0.20 |
| Dimethicone | 1.00 |
| Caprylic/capric triglyceride | 10.00 |
| Butyrospermum parkii (Shea) butter | 2.00 |
| Distearoylisopropyl dimonium methosulfate | 5.00 |
| Glyceryl stearate | 1.00 |
| Cetearyl alcohol | 4.00 |
| Saccharide isomerate (0.5%)* & Water & Citric acid & Sodium citrate | 1.00 |
| Niacinamide | 4.00 |
| Amidinobenzyl benzylsulfonyl D-seryl homophenylalaninamide acetate (0.0003% or 3ppm)* & glycerin & water & citric acid & sodium hydroxide | 0.30 |

| | |
|---|---|
| (*) % by weight based on the total weight of the composition. | |

### Method of manufacture

1. In the main vessel add water, glycerin, saccharide isomerate and water and citric acid and sodium citrate and niacinamide. Stir until solids dissolved and heat to 70-75°C.
2. In a separate vessel weigh out the oil phase (dimethicone, caprylic/capric triglyceride, butyrospermum parkii (Shea) butter, distearoylisopropyl dimonium methosulfate, glyceryl stearate and cetearyl alcohol). Heat to 70-75°C until all molten.
3. Add the oil phase to the water phase and stir together. Homogenise for 5 minutes at 4500 rpm using a Silverson mixer or equivalent.
4. Cool in a water bath with stirring until a temperate of 35°C is reached.
5. Stir in phenoxyethanol, caprylyl glycol and ethylhexylglycerin and amidinobenzyl benzylsulfonyl D-seryl homophenylalaninamide acetate and glycerin and water and citric acid and sodium hydroxide.
6. Dissolve niacinamide in a small amount of water and stir in.
7. Continue to the cool the product to room temperature.
8. Make to weight with water and stir smooth.

### Example 2 - Cationic moisturiser (distearoylisopropyl dimonium methosulfate)

| Material | wt.% |
|---|---|
| Glycerin | 10.00 |
| Water | 65.70 |
| Phenoxyethanol | 0.30 |
| Caprylyl glycol & ethylhexylglycerin | 0.10 |
| Dimethicone | 1.00 |
| Caprylic/capric triglyceride | 10.00 |
| Butyrospermum parkii (Shea) butter | 2.00 |
| Distearoylisopropyl dimonium methosulfate | 2.00 |
| Glyceryl stearate | 1.00 |
| Cetearyl alcohol | 4.00 |
| Panthenol | 0.4 |
| Niacinamide | 3.00 |
| Centella asiatica leaf extract | 0.50 |

### Method of manufacture

1. In the main vessel add water, glycerin and niacinamide. Stir until solids dissolved and heat to 70-75°C.
2. In a separate vessel weigh out the oil phase (dimethicone, caprylic/capric triglyceride, butyrospermum parkii (Shea) butter, distearoylisopropyl dimonium methosulfate, glyceryl stearate and cetearyl alcohol) Heat to 70-75°C until all molten.
3. Add the oil phase to the water phase and stir together. Homogenise for 5 minutes at 4500 rpm using a Silverson mixer or equivalent.
4. Cool in a water bath with stirring until a temperate of 35°C is reached.
5. Stir in phenoxyethanol, caprylyl glycol and ethylhexylglycerin and panthenol.
6. Dissolve centella asiatica leaf extract in a small amount of water and stir in.
7. Continue to the cool the product to room temperature.
8. Make to weight with water and stir smooth.

### Example 3 - Cationic moisturiser (brassicyl valinate esylate)

| Material | wt.% |
|---|---|
| Glycerin | 10.00 |
| Water | 61.35 |
| Phenoxyethanol | 0.40 |
| Caprylyl glycol & ethylhexylglycerin | 0.20 |
| Dimethicone | 1.00 |
| Caprylic/capric triglyceride | 10.00 |
| Butyrospermum parkii (Shea) butter | 2.00 |
| Brassica alcohol & Brassicyl valinate esylate & Brassica glycerides | 6.00 |
| Calcium gluconate | 0.80 |
| Cetearyl alcohol | 4.00 |
| Bisabolol | 1.00 |
| Niacinamide | 3.00 |
| Dipotassium glycyrrhizate | 0.3 |

### Method of manufacture

1. In the main vessel add water, glycerin and calcium gluconate. Stir until solids dissolved and heat to 70-75°C.
2. In a separate vessel weigh out the oil phase (dimethicone, caprylic/capric triglyceride, butyrospermum parkii (Shea) butter, bisabolol, brassica alcohol and brassicyl valinate esylate, and brassica glycerides, glyceryl stearate and cetearyl alcohol). Heat to 70-75°C until all molten.
3. Add the oil phase to the water phase and stir together. Homogenise for 5 minutes at 4500 rpm using a Silverson mixer or equivalent.
4. Cool in a water bath with stirring until a temperate of 35°C is reached.
5. Stir in Phenoxyethanol and caprylyl glycol and ethylhexylglycerin.
6. Dissolve Niacinamide and dipotassium glycyrrhizate in a small amount of water and stir in.
7. Continue to the cool the product to room temperature.
8. Make to weight with water and stir smooth.

### Example 4 - PET test data

A composition containing distearoylisopropyl dimonium methosulfate, 0.4% phenoxyethanol and 0.2% mixture of caprylyl glycol and ethylhexylglycerin was used for a preservative efficacy test (PET), results are shown in Table 1). A PET test pass is required before a product can be marketed. This test ensures that if a product is contaminated with bacteria, mould or yeast then it has enough antimicrobial activity in order to fight off this contamination within a defined time period.

The following strains were used as inoculum. Bacteria: *Escherichia coli* NCIMB 8545 (also referred to as *E. coli), Pseudomonas aeruginosa* ATCC 8626 (also referred to as *P. aeruginosa),* and *Staphylococcus aureus* NCIMB 9518 (also referred to as *S. aureus)* yeast and fungi: *Candida albicans* ATCC 10231 (also referred to as C. *albicans*) and *Aspergillus brasiliensis* ATCC 16404 (also referred to as *A. brasiliensis*).

The inoculum was inoculated into the test sample so that the concentration of the bacterial solution in the test sample consisting of each preparation was 1.5E+06 to 2.3E+06 CFU/mL (for 5 bacteria species). At each sampling point (2, 7, 14 and 28 days) the viable cell count was measured. The test results in Table 4 show the Log10 reduction of the viable cell count at that time point.

As shown in Table 1, the composition tested exhibited antimicrobial effect against the organisms listed in Table 1.

**Table 1: Preservative efficacy test**

| **Organism** | **Inoculum control CFU/mL** | | **Post-inoculation recovery** | | | |
|---|---|---|---|---|---|---|
| | | | **2 days** | **7 days** | **14 days** | **28 days** |
| *Pseudomonas aeruginosa* NCIMB 8626 and *Staphylococcus aureus* NCIMB 9518 | 2.3E+06 | Count CFU/mL | 9.0E+00 | NT* | NT | NT |
| | | Log ₁₀ reduction | 5.41 | | | |
| *Escherichia coli* NCIMB 8545 | 1.5E+06 | Count CFU/mL | 9.0E+00 | NT | NT | NT |
| | | Log ₁₀ reduction | 5.21 | | | |
| *Candida albicans* ATCC 10231 and *Aspergillus brasiliensis* ATCC 16404 | 1.6E+06 | Count CFU/mL | | 1.1E+03 | 9.9E+01 | NT |
| | | Log ₁₀ reduction | | 3.15 | 4.2 | |

| | | | | | | |
|---|---|---|---|---|---|---|
| *NT=Not tested | | | | | | |

### Example 5 - Consumer testing data

Consumer testing of composition containing 6 % brassica alcohol and brassicyl valinate esylate and brassica glycerides and 0.4% phenoxyethanol and 0.2% caprylyl glycol and ethylhexylglycerin included 150 volunteers. Consumers in the study had a range of skin types (including sensitive skin) and age range varies from 25 upwards. Consumers used the product twice a day (morning and evening) for 4 weeks and asked to fill in questionnaires each week.

Following a 4-week trial the numbers of undesirable events (UEs) were recorded in each questionnaire. The total number of UEs obtained were low, some UeEs obtained were due to spots but no consumers withdrew from the trial due to a UE. This shoes that the composition tested was well tolerated on the skin.

## Claims

1. A skincare composition comprising (a) a cationic emulsifier comprising an ester quaternary ammonium cationic emulsifier and/or a non-quaternary ammonium cationic emulsifier, and (b) from 0.01 to 0.6 wt.% of a preservative.

2. The composition of claim 2, wherein the non-quaternary ammonium cationic emulsifier comprises an amino lipid, amidoamine, or combinations thereof.

3. The composition according to claim 1 or claim 2, wherein the cationic emulsifier is present in the compositions in an amount of from 0.05% to 14 % by weight.

4. The composition according to any one of claims 1 to 3, wherein the cationic emulsifier comprises distearoylisopropyl dimonium methosulfate, brassicamidopropyl dimethylamine, brassicyl valinate esylate, distearoylethyl dimonium chloride, bis-(isostearoyl/oleoyl isopropyl) dimonium methosulfate, cannabisamidopropyl dimethylamine, stearamidopropyl dimethylamine, brassicyl isoleucinate esylate or combinations thereof.

5. The composition according to any one of claims 1 to 4, wherein the preservative comprises phenoxyethanol, 2-bromo2-nitropropane-1,3-diol (bronopol, which is available commercially under the trade name Myacide RTM), benzyl alcohol, diazolidinyl urea, imidazolidinyl urea, methyl paraben, phenoxy ethanol, ethyl paraben, propyl paraben, sodium methyl paraben, sodium dehydroacetate, polyhexamethylenebiguanide hydrochloride, sodium benzoate, chlorhexidine digluconate, isothiazolone, sodium propyl paraben or combinations thereof

6. The composition according to any one of claims 1 to 5, wherein the preservative comprises phenoxyethanol.

7. The composition according to any one of claims 1 to 6, wherein the composition comprises from 0.05 to 0.5% by weight of phenoxyethanol.

8. The composition according to any one of claims 1 to 7, wherein one or more of the following applies:
a) the composition further comprises caprylyl glycol, ethylhexylglycerin or combinations thereof;
b) the composition further comprises from 0.01 to 0.3% by weight of a mixture of caprylyl glycol and ethylhexylglycerin;
c) the composition is an emulsion; and
d) the composition is in the form of an oil-in-water emulsion

9. Use of the skincare composition according to any one of claims 1 to 8, for the topical cosmetic application to the skin of a subject.

10. Use of the skincare composition according to any one of claims 1 to 8 for moisturising and/or hydrating the skin of a subject.

11. Use of the skincare composition according to any one of claims 1 to 8 on skin as a non-therapeutic cosmetic treatment for improving skin mildness and/or preventing skin irritation.

12. Use of a composition according to any one of claims 1 to 8 on skin as a non-therapeutic cosmetic treatment for any one or more of the following: improving skin moisture content, hydrating skin, improving the radiance of the skin, improving skin suppleness, reducing skin tightness, improving the healthy visual appearance of the skin, reducing skin dryness, improving the skin barrier function or any combination thereof.

13. A method comprising applying the skincare composition according to any one of claims 1 to 8 to the skin of a subject.

14. A cosmetic method of improving skin mildness and/or reducing skin irritation, the method comprising topically administering the skincare composition according to any one of claims 1 to 8 to the skin of a subject.

15. A cosmetic method of reducing skin dryness and/or moisturising and/or hydrating a skin in need thereof, the method comprising topically administering the skincare composition according to any one of claims 1 to 8 to the skin of a subject.
